# EUROPEAN PATENT APPLICATION

(11) **EP 4 295 865 A2**
(43) Date of publication of application: **27.12.2023**
(21) Application number: 23201742.6
(22) Date of filing: 07.02.2019
(51) Int. Cl.: A61K 47/36

(54) **GEL COMPOSITIONS AND METHODS OF PREPARATION AND USE THEREOF**

(30) Priority: 09.02.2018 US 201862628709 P
(62) Divisional of application: 19707187.1
(71) Applicant: Boston Scientific Scimed, Inc., Maple Grove, MN 55311 (US)
(72) Inventor: The designation of the inventor has not yet been filed
(74) Representative: Vossius & Partner Patentanwälte Rechtsanwälte mbB

(57) **Abstract**

The present invention relates to a medical device comprising_a needle, a reservoir coupled to the needle, and a gel inside the reservoir, the gel comprising gellan gum, a first salt omprising a monovalent cation, a second salt comprising a divalent cation, and water, wherein the gel is biocompatible and injectable through the needle and wherein the gel has a viscosity ranging from 0.004 Pa s to 0.010 Pa·s at a shear rate of 768 s⁻¹.

## Description

### CROSS-REFERENCE TO RELATED APPLICATIONS

This application claims the benefit of priority from U.S. Provisional Application No. 62/628,709, filed on February 9, 2018, which is incorporated by reference herein in its entirety.

### TECHNICAL FIELD

The present disclosure relates generally to compositions for injection to a patient, methods of preparation and use thereof, and devices comprising such compositions.

### BACKGROUND

Various medical procedures are used for diagnosis and/or treatment of tissue. For example, an endoscopic procedure may be performed to take tissue samples from the gastrointestinal (GI) tract or other organ systems for pathological evaluation and/or therapeutic purposes, such as detection and removal of pre-cancerous mucosal tissue or tumors. Yet, removing select portions of tissue from a patient with minimal disturbance to underlying anatomy can be challenging.

In medical procedures such as endoscopic mucosal resection (EMR) and endoscopic submucosal dissection (ESD), a fluid may be injected into tissue to separate different tissue layers to assist in the removal of lesions. For example, a fluid may be injected to separate submucosal tissue from mucosal tissue. The injected fluid generally elevates the target tissue from underlying tissue layers to allow a physician to more easily resect the target tissue. Yet, fluids used for this purpose, such as saline, tend to dissipate within a few minutes, and can require periodic re-injection to ensure the target tissue remains raised throughout the procedure. More viscous injection solutions have been identified, but these alternatives are often costly, difficult to inject, and/or also prone to dissipation/breaking down too soon after injection.

### SUMMARY OF THE DISCLOSURE

The present disclosure includes compositions useful for tissue resection procedures and methods of preparing such compositions. According to some aspects of the present disclosure, the composition may comprise a gel formed from a polysaccharide such as gellan gum, water, a first salt as a source of monovalent cations, and a second salt as a source of divalent cations. The gel may be allowed to set undisturbed, e.g., in a reservoir, to form a continuous, three-dimensional network prior to injection from the reservoir to a patient. The continuous, three-dimensional network may provide for a homogeneous structure of the gel.

The present disclosure includes, for example, a method of preparing a gel for delivery to a target site of a patient, wherein the method comprises: combining gellan gum and water to form a pre-mixture; heating the pre-mixture; adding a first salt comprising a monovalent cation and a second salt comprising a divalent cation to the pre-mixture to form a mixture; introducing the mixture into a reservoir; and cooling the mixture to form the gel inside the reservoir. The gel may be biocompatible and injectable from the reservoir through a needle to the target site. For example, the gel may be a continuous, three-dimensional structure extending across an entire cross-sectional dimension of the reservoir. In some cases, the method may further comprise adding at least one coloring agent to the pre-mixture or the mixture.

According to some aspects, the gel may comprise 0.01% to 2.0% gellan gum by weight, with respect to the total weight of the gel. Alternatively or additionally, the gel may comprise 0.01 % to 0.1% by weight of the second salt, with respect to the total weight of the gel. In some examples, the mixture may have a molar ratio of the monovalent cation to the divalent cation ranging from 5 to 200. In some examples, the monovalent cation of the first salt may be sodium or potassium, and the divalent cation of the second salt may be calcium or magnesium. In at least one example, the first salt may comprise sodium chloride or a hydrate thereof, and the second salt may comprise calcium chloride or a hydrate thereof. In some examples, the gel may have an endotoxin level of 20 endotoxin units (EU) or less.

The gel may have a viscosity ranging from 0.005 Pa·s to 0.050 Pa·s at a shear rate of 130 s⁻¹. Alternatively or additionally, the gel may have a viscosity a viscosity ranging from 0.004 Pa·s to 0.010 Pa·s at a shear rate of 768 s⁻¹. For example, the gel may have a viscosity ranging from 0.015 Pa s to 0.020 Pa s at a shear rate of 130 s⁻¹ and a viscosity ranging from 0.004 Pa s to 0.010 Pa s at a shear rate of 768 s⁻¹. In some examples, the mixture has an osmolality ranging from 240 mOsmol/kg to 340 mOsmol/kg.

In some aspects, the pre-mixture may be heated at a temperature ranging from about 50°C to about 130°C. In some examples, the mixture may have a temperature ranging from about 50°C to about 130°C when introduced into the reservoir. In at least one example, the mixture may be cooled to a temperature below about 50°C before introducing the mixture into the reservoir, and the method may further comprise heating the mixture at a temperature ranging from about 50°C to about 130°C while inside the reservoir. In at least one example, the reservoir may be a barrel of a syringe or the reservoir may be coupled to the needle via a flexible tube.

According to some aspects, the method of preparing a composition for delivery to a target site of a patient may comprise: combining gellan gum and water to form a pre-mixture; heating the pre-mixture; adding a first salt comprising a monovalent cation and a second salt comprising a divalent cation to the pre-mixture to form a mixture, wherein the mixture may have a molar ratio of the monovalent cation to the divalent cation ranging from 5 to 200; heating the mixture; and cooling the mixture to form a homogeneous gel having a continuous, three-dimensional structure. The gel may be biocompatible and injectable from a reservoir through a needle to the target site.

In some examples, the pre-mixture may be heated to a temperature ranging from about 50°C to about 90°C, wherein the mixture may be heated to a temperature greater than the temperature of the pre-mixture. In some examples, the gel may have a viscosity ranging from 0.005 Pa s to 0.050 Pa s at a shear rate of 130 s⁻¹, and a viscosity ranging from 0.004 Pa s to 0.010 Pa s at a shear rate of 768 s⁻¹.

The present disclosure further includes, for example, a medical device that comprises a needle; a reservoir coupled to the needle; and a gel inside the reservoir, the gel comprising: gellan gum; a first salt comprising a monovalent cation; a second salt comprising a divalent cation; and water; wherein the gel may be biocompatible and injectable through the needle, the gel having a viscosity ranging from 0.005 Pa s to 0.05 Pa s at a shear rate of 130 s⁻¹. In some examples, the gel may comprise less than 0.1% by weight of the second salt with respect to a total weight of the gel. In at least one example, the reservoir may be a barrel of a syringe or the reservoir is coupled to the needle via a flexible tube.

In some aspects, the monovalent cation of the first salt may be sodium or potassium, and the divalent cation of the second salt may be calcium or magnesium. In some examples, a molar ratio of the first salt to the second salt in the gel may range from 5 to 200.

In some examples, the gel may have a continuous, three-dimensional structure extending across an entire cross-sectional dimension of the reservoir. In some examples, the gel may comprise 0.01% to 2.0% gellan gum by weight with respect to the total weight of the gel, the gel having an endotoxin level of 20 endotoxin units (EU) or less.

In some aspects, the gel may further comprise at least one coloring agent, such as FD&C Blue 1. In some aspects, the gel further comprises at least one sequestrant, such as calcium citrate, sodium citrate, or calcium phosphate.

### BRIEF DESCRIPTION OF THE FIGURES

The accompanying drawings, which are incorporated in and constitute a part of this specification, illustrate various exemplary aspects of the disclosure, and together with the description serve to explain the principles of the present disclosure.
Figs. 1A-1C show exemplary medical devices in accordance with certain aspects of the present disclosure.
Figs. 2A-2E illustrate an exemplary tissue resection procedure in accordance with certain aspects of the present disclosure.

### DETAILED DESCRIPTION

Particular aspects of the present disclosure are described in greater detail below. The terms and definitions provided herein control, if in conflict with terms and/or definitions incorporated by reference.

As used herein, the terms "comprises," "comprising," or any other variation thereof are intended to cover a non-exclusive inclusion, such that a process, method, composition, article, or apparatus that comprises a list of elements does not include only those elements, but may include other elements not expressly listed or inherent to such process, method, composition, article, or apparatus. The term "exemplary" is used in the sense of "example" rather than "ideal."

As used herein, the singular forms "a," "an," and "the" include plural reference unless the context dictates otherwise. The terms "approximately" and "about" refer to being nearly the same as a referenced number or value. As used herein, the terms "approximately" and "about" should be understood to encompass ± 5% of a specified amount or value.

The present disclosure provides compositions, e.g., gels, for injecting to a patient. The compositions may be injected to a tissue of the patient, e.g., for resecting at least a portion of the tissue. According to some aspects of the present disclosure, the compositions may comprise at least one gelling agent, two or more salts, and water. In some examples, the composition may be or comprise a gel with a desired gel strength and/or viscosity, such as a biocompatible gel suitable for injection (e.g., through a needle). In at least one example, the composition may be a pseudoplastic material that has lower viscosity under shear and higher viscosity at rest.

The gelling agent(s) in the composition may be natural (e.g., natural gums such as vegetable gums and/or microbial gums) or synthetic in origin, and may be anionic, cationic, or neutral. Non-limiting examples of the gelling agents include polysaccharides such as gellan gum, xanthan gum, gum arabic, guar gum, locust bean gum, alginate, and carrageenans.

In at least one example, the gelling agent may comprise gellan gum. As used herein, the term "gellan gum" refers to a polysaccharide (e.g., produced by *Sphingomonas* bacteria), and has a general structure formed of repeating units of four sugars linked together: two residues of D-glucose, one residue of L-rhamnose, and one residue of D-glucuronic acid. The gelling agent may comprise one or more types of gellan gum, e.g., native gellan gum, deacylated gellan gum, or a mixture thereof. The native gellan gum may include two acyl groups (e.g., acetate and glycerate), bound to the glucose residue adjacent to the glucuronic acid residue. These acyl groups may be removed under alkaline conditions to produce deacylated gellan gum, which results in different stability and plasticity properties in comparison to native gellan gum. For example, native gellan gum generally forms softer, more elastic gels with thermoreversibility, whereas deacylated gellan gum generally forms harder, more inelastic gels with higher heat resistance. In at least one embodiment, the composition comprises deacylated gellan gum.

Certain microbial extracts may comprise endotoxins, e.g., lipopolysaccharides from the bacteria that become combined with the polysaccharide structure. In some embodiments, the gelling agent(s) may be chosen to minimize or eliminate the introduction of endotoxins into the composition. In some examples, the gelling agent(s) may have an endotoxin level of 20 endotoxin units (EU) or less, such as from 0 EU to about 20 EU, from 0 EU to about 10 EU, from 0 EU to about 5 EU, from 1 EU to about 20 EU, from about 1 EU to about 10 EU, or from about 1 EU to about 5 EU. Thus, for example, a composition comprising the gelling agent(s) may have an endotoxin level of 20 EU or less, such as from 0 EU to about 20 EU, from 0 EU to about 10 EU, from 0 EU to about 5 EU, from about 1 EU to about 20 EU, from about 1 EU to about 10 EU, or from about 1 EU to about 5 EU. In use, the composition, e.g., gel, may be delivered to a target site of a patient via a suitable medical device (e.g., a syringe or a fluid reservoir coupled to an injection needle). Thus, for example, the medical device may have an endotoxin level of 20 EU or less, such as from 0 EU to about 20 EU, from 0 EU to about 10 EU, from 0 EU to about 5 EU, from about 1 EU to about 20 EU, from about 1 EU to about 10 EU, or from about 1 EU to about 5 EU. Bacterial endotoxin levels may be measured, for example, using the Limulus Amebocyte Lysate (LAL) test. Alternatively or additionally, the gelling agent(s) may be processed to reduce or eliminate the concentration of endotoxins prior to use in the composition disclosed herein. For example, the composition may comprise a microbial-sourced polysaccharide, e.g., xanthan gum, that has been processed to reduce the amount of endotoxins present, such that the resulting composition is pharmaceutically-acceptable and in compliance with the applicable government regulatory standards.

The concentrations of gelling agent(s) in the composition may range from about 0.01% to about 2.0% by weight with respect to the total weight of the composition, such as from about 0.02% to about 1.5%, from about 0.05% to about 1.0%, from about 0.05% to about 0.50%, from 0.05% to about 0.15%, from about 0.10% to about 0.20%, from about 0.15% to about 0.25%, from about 0.20% to about 0.30%, from about 0.25% to about 0.35%, from about 0.30% to about 0.40%, from about 0.35% to about 0.45%, from about 0.40% to about 0.50%, from about 0.1% to about 0.5%, or from about 0.1% to about 0.15% by weight with respect to the total weight of the composition. In at least one example, the total concentration of the gelling agent(s) in the composition may range from about 0.05% to about 0.5% by weight with respect to the total weight of the composition.

According to some aspects of the present disclosure, the composition herein may comprise one or more salts, e.g. physiologically compatible salts. For example, the composition herein may comprise two or more, e.g., two, three, four, five, or more different salts. In at least one example, the composition may comprise two salts.

In some examples, the composition may comprise at least one salt comprising a monovalent cation. Non-limiting examples of such salts include salts comprising sodium and/or potassium cations, e.g., sodium chloride (NaCl), potassium chloride (KCl), sodium dihydrogen phosphate (NaH₂PO₄), potassium hydrogen phosphate (K₂HPO₄), sodium gluconate (C₆H₁₁NaO₇), sodium acetate trihydrate (C₂H₉NaO₅·3H₂O), any hydrates thereof, and any mixture thereof. In at least one example, the salt(s) with a monovalent cation include sodium chloride.

Alternatively or additionally, the composition may comprise a salt comprising a divalent cation. Non-limiting examples of such salts include salts comprising calcium and/or magnesium cations, e.g., calcium chloride (CaCh), magnesium sulfate (MgSO₄), magnesium chloride (MgCh), any hydrates thereof, and any mixture thereof. In at least one example, the salt(s) with a divalent cation comprise calcium chloride or a hydrate thereof, e.g., calcium chloride dihydrate.

In some cases, the composition may comprise at least one salt comprising a monovalent cation and at least one salt comprising a divalent cation. For example, the composition may comprise at least one sodium salt or potassium salt and at least one calcium or magnesium salt, such as, e.g., NaCl and CaCl₂, or NaCl and MgClz, KCl and CaClz, or KCl and MgCh. Further, for example, the composition may comprise at least one salt chosen from sodium chloride, potassium chloride, sodium dihydrogen phosphate, potassium hydrogen phosphate, sodium gluconate, or sodium acetate trihydrate in combination with at least one salt chosen from calcium chloride, magnesium sulfate, or magnesium chloride.

Without intending to be bound by theory, it is believed that salts comprising divalent cations generally provide for stronger gels (e.g., gels with relatively higher gel strength) as compared to salts comprising only monovalent cations. The concentration of each salt of the one or more salts in the composition may range from about 0.01% to about 2.0% by weight with respect to the total weight of the composition, such as from about 0.01% to about 0.50%, from about 0.01% to about 0.20%, from about 0.25% to about 1.0%, from about 0.50% to about 1.5%, from about 0.50% to about 1.0%, or from about 1.0% to about 2.0% by weight with respect to the total weight of the composition, e.g., about 0.25%, about 0.50%, about 0.75%, or about 1.0% by weight with respect to the total weight of the composition. In some examples, the total amount of salt(s) present in the composition may range from about 0.1% to about 4.0% by weight with respect to the total weight of the composition.

In some examples, the composition may comprise a salt with a monovalent cation at a concentration ranging from about 0.10% to about 2.0%, from about 0.10% to about 0.50%, from about 0.25% to about 0.75%, from about 0.50% to about 1.0%, from about 0.75% to about 1.25%, from about 1.0% to about 1.5%, from about 1.25% to about 1.75%, or from about 1.5% to about 2.0% by weight, e.g., about 0.80%, about 0.85%, or about 0.90% by weight, with respect to the total weight of the composition.

Alternatively or additionally, the composition may comprise a salt with a divalent cation at a concentration ranging from about 0.010% to about 0.200%, from about 0.010% to about 0.050%, from about 0.025% to about 0.075%, from about 0.050% to about 0.100%, from about 0.075% to about 0.125%, from about 0.100% to about 0.150%, from about 0.125% to about 0.175%, or from about 0.150% to about 0.200% by weight, e.g., about 0.035%, about 0.040%, about 0.045%, or about 0.050% by weight, with respect to the total weight of the composition. In at least one example, the composition may comprise about 0.85% by weight sodium chloride and about 0.034% by weight calcium chloride, with respect to the total weight of the composition.

The composition herein may comprise two different salts at a ratio providing desired characteristics (e.g., viscosity, three-dimensional-structure, and/or gel strength) for the compositions. In some examples, the composition may comprise a first salt (e.g., a salt with a monovalent cation) and a second salt (e.g., a salt with a divalent cation) at a molar ratio ranging from about 5 to about 200, from about 50 to about 150, from about 80 to about 120, from about 5 to about 50, from about 25 to about 75, from about 50 to about 100, from about 75 to about 125, from about 100 to about 150, from about 125 to about 175, or from about 150 to about 200. In at least one example, the composition may comprise a sodium chloride and a calcium chloride at a molar ratio ranging from about 5 to about 50, from about 10 to about 20, or from about 15 to about 35. For example, the composition may comprise sodium chloride and calcium chloride having a molar ratio (sodium chloride : calcium chloride) of about 15, 16, 17, 18, 19, 20, 21, 22, 23, 24, or 25.

In some examples, the composition may comprise a physiologically compatible saline solution, such as, e.g., a sodium chloride solution. For example, the composition may comprise a 0.9% wt. sodium chloride solution, e.g., providing sodium cations to assist in formation of the three-dimensional solid gel network. In some cases, the composition may be isotonic. For example, the saline solution may have an appropriate concentration of monovalent and/or divalent cations such that the compositions are isotonic with tissue fluids and/or blood. Other physiologically compatible solutions comprising suitable ionic concentrations may be used to provide for isotonicity.

The composition herein may further comprise one or more additives. According to some aspects of the present disclosure, the composition may comprise one or more biocompatible dyes or coloring agents. In some examples, the dye(s) or colorant(s) may allow for identification of the submucosal tissue plane upon injection into tissue, e.g., to determine the amount of tissue to be removed and/or assess the risk of perforation. Examples of the dye(s) or colorant(s) include, but are not limited to, brilliant blue (e.g., Brilliant Blue FCF, also known as FD&C Blue 1), indigo carmine (also known as FD&C Blue 2), indigo carmine lake, FD&C Blue 1 lake, methylene blue (also known as methylthioninium chloride), or a mixture thereof. Using FD&C Blue 1, in particular, as a coloring agent has been found to maintain the color of the composition over time as compared to other coloring agents such as methylene blue. Maintaining the color of the composition may allow for better identification of target tissue during a medical procedure.

The coloring agent(s) in the composition may have a concentration ranging from about 0.0001% to about 0.0100%, from about 0.0001% to about 0.0050%, from about 0.0005% to about 0.0030%, from about 0.0001% to about 0.0020%, from about 0.0010% to about 0.0030%, from about 0.0020% to about 0.0040%, from about 0.0030% to about 0.0050%, from about 0.0040% to about 0.0060%, from about 0.0050% to about 0.0070%, from about 0.0060% to about 0.0080%, from about 0.0070% to about 0.0090%, or from about 0.0080% to about 0.0100% by weight, with respect of the total weight of the composition. In some examples, the coloring agent(s) in the composition may have a concentration ranging from about 0.0005% to about 0.0030% by weight with respect of the total weight of the composition. In at least one example, the coloring agent(s) in the composition may have a concentration of about 0.001% by weight in respect of the total weight of the composition.

As used herein, the term "sequestrant" refers to any substance capable of forming a complex with at least one metal (e.g., alkali metal, alkaline-earth metal, or transition metal) ion. Without intending to be bound by theory, it is believed that the sequestrant(s) may facilitate formation of a gel with desired characteristics (e.g., 3D structure, gel strength, and/or viscosity). In some cases, for example, incorporating a sequestrant may allow for formation of a gel without a heating step, e.g., forming a gel at or near room temperature (about 20°C to about 25°C). For example, the sequestrant(s) may allow for hydration of the gelling agent at or near room temperature. Examples of sequestrants suitable for the compositions herein may include, but are not limited to, calcium citrate, sodium citrate, calcium phosphate, and any combinations thereof.

Any other suitable types of biocompatible agents may also be included in the composition, e.g., to adjust the pH and/or tonicity of the composition as appropriate for injection into tissue. For example, the composition may comprise one or more stabilizers and/or preservatives. According to some aspects of the present disclosure, the composition may comprise an additive such as epinephrine to limit superficial bleeding. The composition may include one or more additives that improve visualization of diseased tissue or that have a therapeutic effect. For example, the additive may be pharmaceutically active, e.g., actively fighting cancerous cells.

The composition may have a viscosity suitable for injection. As mentioned above, in some examples, the composition may be pseudoplastic. Pseudoplasticity generally refers to the property of decreasing in viscosity upon the application of shear force. Thus, for example, the composition may have a higher viscosity at rest or under low shear conditions (e.g., while stored in a container) than while under high shear conditions (e.g., during loading into and/or injection through a needle). Examples of materials that may exhibit pseudoplasticity include gellan gum and xanthan gum, among other types of polysaccharides.

For example, the composition may have a first viscosity at a first shear rate and a second viscosity at a second shear rate, where the first viscosity is lower than the second viscosity when the first shear rate is higher than the second shear rate. The viscosity of the composition may be measured by a viscometer, e.g., a rheometer. In some examples, the composition may have a viscosity ranging from about 0.001 pascal-second (Pa s) to about 0.100 Pa s at a shear rate of 130 s⁻¹, such as, e.g., from about 0.005 Pa s to about 0.050 Pa s, from about 0.010 Pa s to about 0.050 Pa s, from about 0.010 Pa s to about 0.030 Pa s, from about 0.010 Pa s to about 0.020 Pa s, from about 0.020 Pa s to about 0.030 Pa s, or from about 0.020 Pa s to about 0.040 Pa s at a shear rate of 130 s⁻¹. Thus, for example, the composition may be or comprise a gel having a viscosity of about 0.005 Pa·s, about 0.006 Pa s, 0.008 Pa s, about 0.010 Pa s, about 0.011 Pa s, about 0.012 Pa s, about 0.013 Pa s, about 0.014 Pa·s, about 0.015 Pa·s, about 0.016 Pa·s, about 0.017 Pa·s, about 0.018 Pa·s, about 0.019 Pa·s, about 0.020 Pa·s, about 0.022 Pa·s, about 0.024 Pa·s, about 0.026 Pa·s, about 0.028 Pa s, about 0.030 Pa s, about 0.032 Pa s, about 0.034 Pa s, about 0.036 Pa s, about 0.038 Pa·s, about 0.040 Pa·s, about 0.042 Pa·s, about 0.044 Pa·s, about 0.046 Pa·s, about 0.048 Pa s, or about 0.050 Pa s at a shear rate of 130 s⁻¹. In at least one example, the composition may have a viscosity greater than 0.0050 Pa·s at a shear rate of 130 s⁻¹, e.g., a viscosity ranging from about 0.005 Pa s to about 0.050 Pa s, at a shear rate of 130 s⁻¹. In at least one example, the composition may have a viscosity greater than 0.010 Pa s at a shear rate of 130 s⁻¹, e.g., a viscosity ranging from about 0.010 Pa s to about 0.030 Pa s, at a shear rate of 130 s⁻¹.

Alternatively or additionally, the composition may have a viscosity ranging from about 0.001 Pa s to about 0.050 Pa s at a shear rate of 768 s⁻¹, such as, e.g., from about 0.002 Pa s to about 0.030 Pa s, from about 0.003 Pa s to about 0.020 Pa s, from about 0.004 Pa·s to about 0.010 Pa·s, from about 0.004 Pa·s to about 0.006 Pa·s, from about 0.005 Pa·s to about 0.007 Pa s, from about 0.006 Pa s to about 0.008 Pa s, from about 0.007 Pa s to about 0.009 Pa s, or from about 0.008 Pa s to about 0.01 Pa s at a shear rate of 768 s⁻¹. Thus, for example, the composition may be or comprise a gel having a viscosity of about 0.003 Pa s , about 0.004 Pa s, about 0.005 Pa s, about 0.006 Pa s, about 0.007 Pa s, about 0.008 Pa s, about 0.009 Pa s, or about 0.010 Pa s at a shear rate of 768 s⁻¹. In at least one example, the composition may have a viscosity less than 0.010 Pa s at a shear rate of 768 s⁻¹, e.g., a viscosity ranging from about 0.005 Pa s to about 0.009 Pa s at a shear rate of 768 s⁻¹ In at least one example, the composition may have a viscosity ranging from about 0.004 Pa s to about 0.010 Pa s at a shear rate of 768 s⁻¹. Further, for example, the composition may have a viscosity ranging from about 0.010 Pa·s to about 0.030 Pa·s, e.g., about 0.017 Pa·s at a shear rate of 130 s⁻¹ and a viscosity ranging from about 0.004 Pa·s to about 0.010 Pa·s, e.g., about 0.007 Pa s, at a shear rate of 768 s⁻¹.

The present disclosure also provides medical devices comprising the composition herein. The medical devices may be used for injecting the composition to a tissue in a patient, e.g., for resecting at least a portion of the tissue.

According to some aspects of the present disclosure, the medical device may comprise one or more reservoirs. The reservoir may serve as a container for the composition herein. Suitable reservoirs may include, for example, syringes (e.g., a syringe barrel compatible with a manual or automatic injection system), flexible pouches such as a plastic bag, and other fluid containers configured for use with a suitable injection needle. Exemplary materials suitable for the reservoir include, but are not limited to, cyclic olefin copolymer, cyclic olefin polymer, polypropylene, polycarbonate, polyvinyl chloride, and glass.

The medical device herein may comprise one or more needles. In some examples, the reservoir of the medical device may be directly coupled to the needle(s), e.g., via a Luer adapter or other suitable connection, or may be indirectly coupled to the needle(s) via a flexible tube, such as a catheter. Non-limiting examples of needles coupled with a reservoir via a flexible tube include Interject^{™} sclerotherapy needles by Boston Scientific. In some examples, the needle may be a hypodermic needle, and may range from a size of 7 gauge (4.57 mm outer diameter (OD), 3.81 mm inner diameter (ID)) to 33 gauge (0.18 mm OD, 0.08 mm ID), e.g., a size of 16 gauge (1.65 mm OD, 1.19 mm ID), 21 gauge (0.82 mm OD, 0.51 mm ID), 22 gauge (0.72 mm OD, 0.41 mm ID), 23 gauge (0.64 mm OD, 0.33 ID), or 24 gauge (0.57 mm OD, 0.31 mm ID). Exemplary materials for the needle include, but are not limited to, metals and metal alloys, such as stainless steel and Nitinol, and polymers. The distal tip of the needle may be sharpened, and may have a beveled shape. The proximal end of the needle may include a suitable fitting/adaptor (e.g., a Luer adapter) for engagement with a syringe or other reservoir. In some examples, the needle may include an elongated tube or catheter between the needle tip and the proximal fitting/adapter.

Further disclosed herein are methods of making the compositions and the devices. In general, the methods may comprise combining the gelling agent(s) and water to form a pre-mixture, heating the pre-mixture, adding the salt(s) to the pre-mixture to form a mixture, and introducing the mixture to the reservoir. In some examples, the mixture may form a gel while inside the reservoir.

In at least one example, the method may include one or more steps of hydrating one or more gelling agents (e.g., gellan gum) by combining the gelling agent(s) with water (e.g., optionally referred to herein as a pre-mixture); heating the hydrated gelling agent(s); adding at least two different salts (e.g., a salt comprising a monovalent cation and a salt comprising a divalent cation) to the hydrated gelling agent(s) to form a mixture; introducing the mixture of gelling agent(s) water, and salts into a reservoir; and cooling the mixture to form a gel before or after introducing the mixture into the reservoir. In another example, the method may include one or more steps of hydrating one or more gelling agents (e.g., gellan gum) by combining the gelling agent(s) with water; heating the hydrated gelling agent(s); adding at least two different salts (e.g., a salt comprising a monovalent cation and a salt comprising a divalent cation) to the hydrated gelling agent(s) to form a mixture, optionally wherein the mixture has a molar ratio of the monovalent cation to the divalent cation ranging from5 to 200; heating the mixture; and cooling the mixture to form a homogeneous gel having a continuous, three-dimensional structure. In these examples, the resulting gel may be biocompatible and injectable from a reservoir, e.g., a syringe barrel, IV bag, or other suitable reservoir for a medical composition, through a needle to a target site of a patient.

According to some aspects of the present disclosure, the pre-mixture of the gelling agent(s) and water (e.g., hydrated gelling agent(s)) may be heated before the salt(s) are added. In some examples, the pre-mixture may be heated at a temperature ranging from about 50°C to about 130°C, such as from about 70°C to about 130°C, from about 80°C to about 125°C, from about 90°C to about 115°C, from about 95°C to about 105°C, or from about 70°C to about 90°C, e.g., a temperature of about 50°C, about 55°C, about 60°C, about 65°C, about 70°C, about 75°C, about 80°C, about 85°C, about 90°C, about 95°C, about 100°C, about 105°C, about 110°C, about 115°C, about 120°C, about 125°C, or about 130°C. In some examples, a temperature less than about 90°C, e.g., ranging from about 50°C to about 60°C or ranging from about 70°C to about 85°C, may be used. In some examples, the pre-mixture may be heated to boiling, e.g., a temperature ≥ 100°C. The pre-mixture may be heated for an amount of time sufficient to hydrate the gelling agent(s). For example, the pre-mixture of gelling agent(s) and water may be heated for a time ranging from about 1 minute to about 90 minutes, from about 5 minutes to about 60 minutes, from about 15 minutes to about 45 minutes, or from about 20 minutes to about 30 minutes, e.g., about 15 minutes, about 20 minutes, about 30 minutes, or about 45 minutes.

When a sequestrant is used, the method may comprise combining one or more gelling agents with at least one sequestrant and water in order to hydrate the gelling agent(s) and form a pre-mixture. In some examples, the pre-mixture comprising the gelling agent(s), sequestrant(s), and water is not heated.

The methods herein may comprise adding one or more salts to the hydrated gelling agent(s) to form a mixture. For example, the salt(s) may be added after the pre-mixture is heated to at least 50°C. In at least one example, the salt(s) may be added after the pre-mixture is heated for about 10 minutes and/or when the pre-mixture reaches a temperature of at least 70°C. In some examples, the salt(s) may be added to the pre-mixture during heating, and the resulting mixture may continue to be heated under the same conditions (e.g., at the same temperature, with stirring, etc.). Alternatively, the heated pre-mixture may be cooled before the salt(s) is added, or the pre-mixture may be at a temperature below 50°C, such as at or near room temperature, such as when a sequestrant is used as discussed above.

In some examples, the methods herein may comprise adding a first salt with a monovalent cation, a second salt with a divalent cation, or a combination thereof. When adding both the first and the second salts, the two salts may be mixed first and then added to the pre-mixture. Alternatively, the first and the second salts may be added to the pre-mixture sequentially, e.g., the first salt followed by the second salt, or vice versa.

According to some aspects of the present disclosure, the methods herein may further comprise adding one or more coloring agents, one or more sequestrants, and/or one or more other additives to form the mixture. For example, the methods may comprise adding one or more coloring agents after the one or more salts are added to form the mixture. Alternatively or additionally, in another example, the methods may comprise adding one or more coloring agents before the one or more salts are added to form the mixture.

The resulting mixture may be physiologically compatible, e.g., having electrolyte levels, osmolality, and pH suitable for injection into a patient once the mixture forms a gel. In some examples, the mixture may have an osmolality ranging from about 240 mOsmol/kg to about 340 mOsmol/kg (e.g., 290 mOsmol/kg ± 50 mOsmol/kg), such as from about 250 mOsmol/kg to about 320 mOsmol/kg, or from about 280 mOsmol/kg to about 300 mOsmol/kg. In at least one example, the mixture may have an osmolality of about 290 mOsmol/kg.

Alternatively or additionally, the methods may comprise adjusting the pH of the hydrated gelling agent(s) (pre-mixture) and/or the mixture. The pH of the pre-mixture and/or mixture may be adjusted using an acid (e.g., hydrochloric acid) or a base (e.g., sodium hydroxide), or with other substances providing for a biocompatible composition.

Without intending to be bound by theory, with respect to gelling agents that are polysaccharides like gellan gum, it is believed that the polysaccharide molecules may undergo a coil to double-helix transition with decreasing temperature, which may lead to gel formation, e.g., depending on the ionic strength and pH of the solution. For example, gellan gum coil molecules may form double helices with a reduction in temperature, and these helices may aggregate to form junction zones, resulting in gelation. In water, at low ionic strength and neutral pH, aggregation of the helices may be impeded by electrostatic repulsion between negatively charged carboxylic groups on the gellan molecules. The addition of salt(s) and/or the adjusting (e.g., reducing) of pH may decrease intermolecular repulsion between the helices, thereby enhancing junction zone formation, and consequently, the gel strength. The addition of the salt(s) therefore may facilitate physical cross-linking in an aggregation-like process to form a continuous, three-dimensional gel network. This continuous, three-dimensional network may provide for a solid or quasi-solid gel capable of maintaining its three-dimensional form even when inverted while in an open container.

Alternatively or additionally, after addition of the salt(s), the resulting mixture may be heated. The mixture may be heated under the same conditions as the pre-mixture was heated. Alternatively, the mixture may be heated under different conditions than the pre-mixture (e.g., including when the pre-mixture is not heated, such as when a sequestrant is used). For example, the mixture may be heated at a temperature higher than the pre-mixture. In some examples, the mixture may be heated at a temperature ranging from about 50°C to about 130°C, such as from about 70°C to about 130°C, from about 80°C to about 125°C, from about 90°C to about 115°C, from about 95°C to about 105°C, or from about 70°C to about 90°C , e.g., a temperature of about 50°C, about 55°C, about 60°C, about 65°C, 70°C, about 75°C, about 80°C, about 85°C, about 90°C, about 95°C, about 100°C, about 105°C, about 110°C, about 115°C, about 120°C, about 125°C, or about 130°C. In some examples, a minimum temperature ranging from about 50°C to about 60°C may be used. In some examples, a minimum temperature ranging from about 70°C to about 85°C may be used. In some examples, the mixture may be heated to boiling, e.g., a temperature ≥ 100°C. In some examples, the mixture may be heated for a time ranging from about 5 minutes to about 90 minutes, from about 10 minutes to about 60 minutes, from about 15 minutes to about 45 minutes, or from about 20 minutes to about 30 minutes, e.g., about 15 minutes, about 20 minutes, about 30 minutes, or about 45 minutes. The mixture and/or pre-mixture may be heated with constant or intermittent stirring, e.g., with a magnetic stirrer or other appropriate mixing equipment.

The methods herein may further comprise cooling the mixture. When heated above certain temperature, the mixture may form a low viscosity fluid. When cooled below a certain temperature or range of temperatures, the viscosity and/or the gel strength of the mixture may increase, and set into a gel. In at least one example, the mixture may be cooled to form a homogenous gel that has a continuous, three-dimensional structure. In some examples, the mixture may be cooled to a temperature at or below about 55°C or about 50°C, e.g., about room temperature (about 20°C to about 25°C). The cooling may be performed by allowing the mixture to sit undisturbed at room temperature (e.g., from about 20 °C to about 25 °C) or a temperature below room temperature (e.g., at about 4°C) for a period of time.

In some examples, the mixture may be allowed to cool without stirring or other agitation. In such cases, the mixture may form a substantially homogeneous gel, e.g., a continuous solid. Thus, for example, the resulting gel may have a substantially continuous, three-dimensional, solid or quasi-solid gel network, as opposed to an agglomerate of gel particles or a colloid mixture. Alternatively or additionally, the mixture may be agitated as it cools, e.g., by constant or intermittent stirring. In such cases, the agitation may at least partially disrupt the structure of the gel, e.g., breaking apart the three-dimensional network to form individual gel particles or gel fragments. Alternatively or additionally, the structure of the gel may be at least partially disrupted after the composition cools, e.g., by stirring, shaking, and/or transferring the composition between containers.

According to some aspects of the present disclosure, the methods herein may comprise introducing the mixture (e.g., any of the mixtures described above) into a reservoir of a medical device (e.g., a storage container of an injection device or an injection system). The mixture may be introduced to the reservoir after being heated, e.g., at a temperature ranging from about 70°C to about 130°C, such as a temperature ranging from about 90°C to about 110°C. For example, the mixture may be heated and cooled in an initial storage container, such as a vial, and subsequently transferred into a suitable reservoir from which the mixture may be injected into a patient. In such cases, the mixture may be agitated as it cools in the initial container to form an agglomeration of smaller gel particles or gel-like fluid.

Alternatively or additionally, the mixture may be heated after it is introduced into the reservoir. For example, the mixture may be agitated, sheared, extruded, or otherwise broken up after cooling and housed in the storage container. The agglomeration of gel particles or gel-like fluid may be subsequently mixed with additional liquid components (e.g., other viscous agents, including viscous forms of gellan gum) after the gel has set. The mixture then may be transferred from the storage container to a suitable reservoir, heated, and subsequently allowed to cool to set into a homogeneous gel inside the reservoir. The mixture then may be injected directly from the reservoir through a needle to the target site of a patient. According to some aspects, the gel may be subjected to minimal shear forces and/or other forces prior to injection into a patient. The viscosity of the mixture while set into gel form within the reservoir, prior to injection, may depend on the properties of the gelling agent(s) and/or the concentration of the gelling agent(s) relative to other components of the mixture.

The mixture subsequently may be allowed to cool and increase in viscosity to set into a homogeneous, solid or quasi-solid gel while inside the reservoir. In some cases, the mixture may be re-heated after its introduction into the reservoir and subsequently allowed to cool to set into its final solid or quasi-solid, three-dimensional gel form. For example, the mixture may undergo one or more heating/cooling cycles once introduced into the reservoir. According to some aspects, for example, the mixture may be heated twice by initially heating the mixture of gelling agent(s), salt(s), and water (e.g., to ensure hydration), and then subsequently heating the mixture after introducing the mixture into the reservoir from which it will be injected, allowing it to cool and set into a gel with a continuous, three-dimensional structure. According to some aspects of the present disclosure, the mixture is not transferred from the reservoir to any other container prior to injection from the reservoir directly to the target site of a patient.

The methods herein may further include sterilizing the mixture. For example, the mixture may be autoclaved while inside the reservoir by heating the mixture at or to a temperature of about 121°C. Alternatively or additionally, the mixture may be sterilized via gamma irradiation or by electron beam after its introduction into the reservoir.

Without intending to be bound by theory, it is believed that the application of various forces (e.g., shear force, compression force, stress, friction, etc.) may affect the continuity of the three-dimensional gel network, which in turn may impact its properties prior to use in medical procedures such as tissue resection. For example, transferring the composition between containers prior to injection may lead to shearing of the three-dimensional structure of the gel when ultimately injected into a patient. In some cases, this may limit the effectiveness of the composition, e.g., by limiting the ability of the gel to separate tissue layers and/or reducing the amount of time the gel remains within the tissue (e.g., within submucosal tissue) prior to diffusion or absorption into the tissue.

According to some aspects of the present disclosure, the compositions herein, e.g., the compositions prepared by the methods herein may have sufficient strength, e.g., gel strength, to withstand the forces and thus minimizing the effects of the forces on the continuity of the three-dimensional gel network. In the meantime, the composition with sufficient strength may have a viscosity suitable for injection, e.g., a viscosity that does not render the composition stuck in the reservoir or the needle of the medical device. Alternatively or additionally, the composition may be prepared such that it sets into a continuous, three-dimensional gel network while the composition is inside a reservoir of the medical device, such as an injection device. The composition may form a substantially homogeneous gel solid or quasi-solid in the reservoir without the need to disrupt the gel structure by transferring between storage containers.

Thus, the composition may maintain its three-dimensional structure until the gel is injected through a needle, whereupon the structure may form fragments of the original continuous, three-dimensional network. Those gel fragments may have a diameter corresponding to the diameter of the injection needle, such that the fragments are as large as possible in-vivo to retain as much of the three-dimensional structure of the gel as possible. Injection of these larger-sized particles or fragments is believed to increase the amount of time the gel remains within the tissue.

Further disclosed herein are methods of resecting at least a portion of a tissue from a subject (e.g., a human patient). The methods may comprise injecting the composition described herein to a tissue of the subject and resecting at least a portion of the tissue from the patient. In some example, the composition may be a submucosal lifting agent.

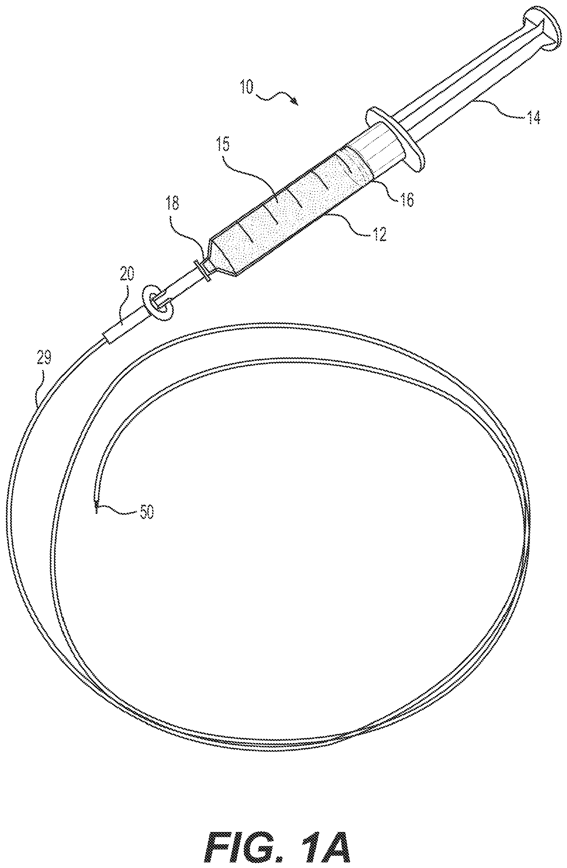

Fig. 1A illustrates an exemplary syringe 10 providing a reservoir for a gel composition as discussed above. The syringe 10 may comprise a barrel 12, a plunger 14, and one or more stoppers 16. The composition 15 may be prepared as discussed above and allowed to set into a solid gel with a continuous, three-dimensional structure across the diameter of the barrel 12. The barrel 12 may include a Luer adapter (or other suitable adapter/connector), e.g., at the distal end 18 of the barrel 12, for attachment to an injection needle 50 via a flexible catheter 29. The proximal end of the catheter 29 may include a suitable connection 20 for receiving the barrel 12. In other examples, the barrel 12 may be directly coupled to the injection needle 50. The syringe barrel 12 may serve as a reservoir, containing a gel composition 15 for injection through the needle 50.

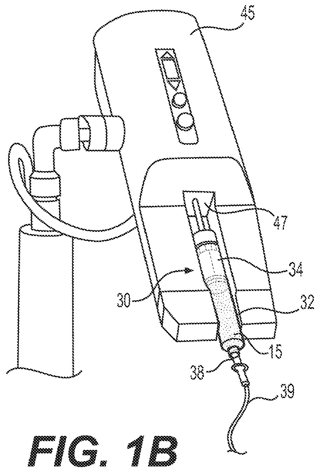

Fig. 1B illustrates an exemplary syringe 30 for use with an automatic injection system 45. The syringe 30 may include any of the features of the syringe 10 of Fig. 1A, e.g., a barrel 32, a plunger 34, and a Luer adapter (or other suitable adapter/connector) at the distal end 38 of the barrel 32. A composition 15 may be prepared as discussed above and allowed to set into a gel in the barrel 32, and the syringe 30 may be inserted into a channel 47 of the injection system 45 for automatic control over the amount of gel injected. The distal end 38 of the syringe 30 may be coupled to an injection needle (e.g., similar to injection needle 50 of Fig. 1A) via a catheter 39. According to some aspects of the present disclosure, the plunger 34 may form part of the injection system 45 and the barrel 32 may be a separate component, e.g., a replaceable cartridge, to be connected to the injection system 45. For example, the composition 15 may be prepared in the barrel 32 as a replaceable cartridge having a proximal attachment compatible with a plunger component of the injection system 45.

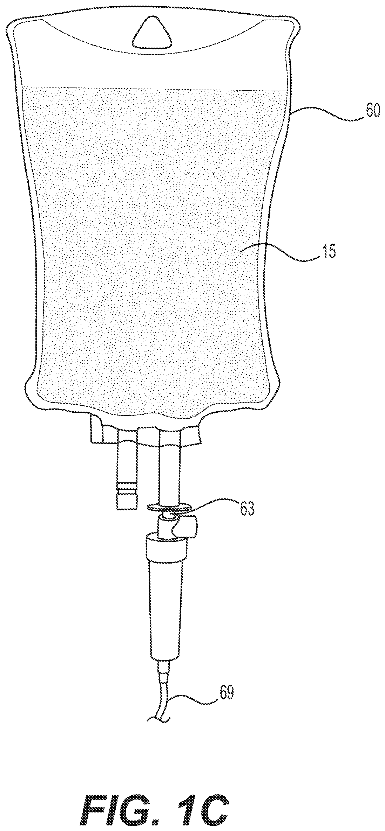

Fig. 1C illustrates an exemplary reservoir 60 according to some aspects of the present disclosure. The reservoir 60 may be provided by a flexible pouch or bag, such as an IV bag. A composition 15 may be prepared as discussed above and allowed to set into a gel in the reservoir 60. The reservoir 60 may be sterile, and may comprise a plastic material such as polyvinyl chloride (PVC) (e.g., with a plasticizer such as bis(2-ethylhexyl) phthalate (DEHP)) or a non-PVC plastic material. The pouch may include a Luer adapter 63 for attachment to a catheter 69 and/or needle (having any suitable gauge size, as described above) for injecting the composition 15 into a patient. The reservoir 60 may be compressible, e.g., to allow for delivery of the composition through the catheter 69 and/or needle by compression of the reservoir 60.

Reservoirs and injection methods other than those illustrated in Figs. 1A-1C may be used in according with the present disclosure. For example, the composition may be housed in a reservoir coupled to a fluid channel and/or needle that forms part of an electrocautery device or system. Thus, a physician may inject the composition through the fluid channel while simultaneously or subsequently operating other portions of the device or system, such as an electrocautery knife or snare.

The amount of force required to move the composition through a needle aperture (generally described as "peak load" force) may depend on the viscosity of the composition, the dimensions of the needle (inner diameter, outer diameter, and/or length), and/or the material(s) from which the needle is formed. For example, a greater amount of force may be applied to inject the composition through a 33 gauge needle in comparison to a 7 gauge needle. Additional factors that may affect the amount of force applied to inject the composition may include the dimensions of a catheter (inner diameter, outer diameter, and/or length) connecting the reservoir to the needle. Suitable peak loads for injection with one or two hands may range from about 5 lbf to about 25 lbf, such as from about 10 lbf to about 20 lbf, e.g., about 15 lbf. The loads measured for a given gel concentration may vary for different needles and flow rates.

According to some aspects of the present disclosure, the size of the needle may be chosen based on the viscosity and/or components of the composition, or vice versa. Further, the dimensions of the catheter tubing (inner diameter, outer diameter, and/or length), if any, may affect the types and amount of force applied to the composition during injection. These parameters may be taken into consideration according to the properties of the composition and the needs of the patient. According to some aspects of the present disclosure, the size of the needle may be 23 gauge or 25 gauge. In some cases, a larger size of 20 gauge, 21 gauge, or 22 gauge may be used to inject the compositions herein.

The compositions herein may be used in various medical procedures, including tissue resection procedures of the GI system, the respiratory system, and/or the genitourinary system. The tissue resected in such medical procedures may comprise diseased or injured tissue, non-diseased tissue, or a combination thereof. Exemplary tissue resection procedures include endoscopic mucosal resection (EMR) and endoscopic submucosal dissection (ESD). In these procedures, an endoscope is typically inserted into the patient's esophagus and advanced through the GI system to reach the target site in the esophagus, stomach, or intestine. EMR is typically used for removal of tissue smaller than 2 cm in diameter, e.g., to biopsy tissue or to remove injured or diseased tissue (e.g., a cancerous lesion), while ESD is typically used for removal of larger lesions.

In some aspects, a continuous solid or quasi-solid gel composition may be prepared as discussed above and injected between two layers of tissue, e.g., injected into submucosal tissue between an upper mucosal layer and lower muscularis propria layer at a target treatment site. The composition may be injected within the submucosal space (submucosal layer) under a portion of tissue, whereupon the injected gel may cause the mucosal tissue to separate from the muscularis propria layer, elevating the mucosal tissue layer. A suitable cutting device, e.g., an electrocautery cutting device such as a knife, snare, scissors, or forceps, may then be used to remove the portion of tissue. For removal of larger portions of tissue (e.g., via ESD), the composition may be injected under the portion of tissue, wherein the gel elevates the upper layer of tissue from the lower layer. The cutting device then may be used to make an incision around the portion of tissue and remove it. The composition may be injected in the submucosal layer to assist in removing additional portions of tissue.

In some aspects, the composition may maintain separation of the tissue layers throughout the entire resection procedure. A portion of the gel composition may be removed via the resection process. Following tissue resection, remaining portions of the gel composition may be flushed from the site with water or saline, or may naturally diffuse into the tissue.

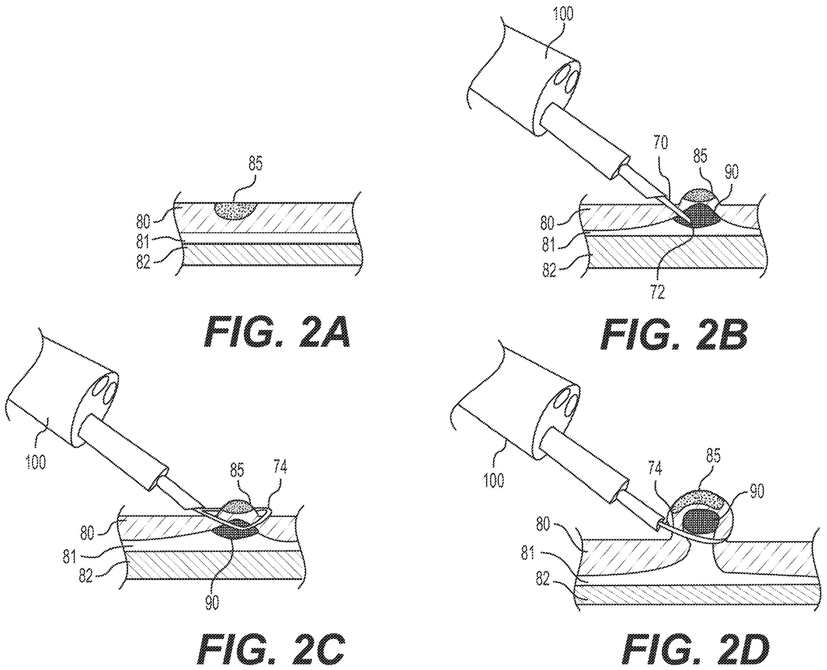

Figs. 2A-2E illustrate an exemplary resection procedure according to some aspects of the present disclosure. For example, the procedure may be EMR or ESD as discussed above, or any other suitable medical procedure for resecting tissue. Fig. 2A shows a cross-sectional view of two portions of tissue or tissue layers 80, 82, which may be separated by a middle layer 81 of tissue (such as, e.g., upper mucosal and lower muscularis propria layers separated by a middle submucosal tissue layer). One or both of the portions of tissue 80, 82 may include a section of tissue 85 targeted for removal. For example, the section of tissue 85 may comprise injured or diseased tissue, or may comprise tissue targeted for biopsy and subsequent analysis. In the example of Fig. 2A, the section of tissue 85 is located toward the tissue surface, however, the devices and compositions disclosed herein may be used to remove tissue from inner tissue layers.

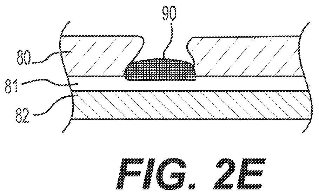

As shown in Fig. 2B, an endoscope 100 defining one or more lumens (e.g., three lumens as shown) may be used to deliver a needle 70 to the treatment site. The needle 70 may have a hollow lumen and a sharp, beveled tip 72 for piercing the tissue surface such that the needle tip 72 is within the middle layer 81 between the upper and lower portions of tissue 80, 82. The needle lumen may be in communication with a fluid reservoir, such as a syringe or other reservoir containing a continuous, solid gel composition 90 prepared as discussed above. The syringe may be used to inject the composition 90 into the middle layer 81 between the portions of tissue 80, 82 to form a cushion or bleb of gel, as shown in Fig. 2B. Once the composition 90 is injected, the volume of the gel 90 may cause the upper and lower portions of tissue 80, 82 to separate, such that the section of tissue 85 may be elevated from underlying tissue. An electrocautery snare 74 or other cutting device 74 (such as, e.g., an electrocautery knife, scissors, or forceps, among other suitable cutting devices) may be used to cut and remove the section of tissue 85, as shown in Figs. 2C and 2D. Once the section of tissue 85 is removed, as shown in Fig. 2E, a portion of the gel 90 may naturally diffuse into one or more of the tissue layers 80, 81, 82.

Other aspects and embodiments of the present disclosure will be apparent to those skilled in the art from consideration of the specification and practice of the embodiments disclosed herein. While certain features of the present disclosure are discussed within the context of exemplary tissue resection procedures, the compositions, systems, and methods may be used for other medical procedures according to the general principles disclosed.

### EXAMPLES

The following examples are intended to illustrate the present disclosure without, however, being limiting in nature. It is understood that the present disclosure encompasses additional aspects and embodiments consistent with the foregoing description and following examples.

### Example 1

This example describes an exemplary procedure for preparing a gel composition according to an embodiment of the present disclosure. Specifically, 1.125 g of Kelcogel CG-LA gellan gum and 0.01g of FD&C Blue 1 were added to 899 g of water to form a pre-mixture. The pre-mixture was heated with stirring until it reached 75°C. Then, 8.1 g of sodium chloride and 0.132 g of calcium chloride dihydrate were added and incorporated to the heated pre-mixture to form a mixture. The mixture was then introduced into the barrel of a 10cc syringe. The filled syringe was sterilized by autoclaving at about 122°C for about 30 minutes.

The viscosity in the syringe was tested by cone and plate rheometer (DHR-1 by TA Instruments) using a 60mm cone with 01:01:01 degree:minute:second cone angle, and a truncation gap of 28µm. A minimum of 1.00379mL of the test sample was injected through a 23 gauge orifice onto the Peltier plate and the temperature of the plate dwelt at 37°C for a minimum of 60 seconds before running the test. The cone then dwelt at each shear rate for a minimum of 30 seconds with viscosity data recorded once every second at which point an average of the values collected for each shear rate was used as the viscosity at that shear rate.

The resulting mean viscosity at a shear rate 130 s⁻¹ was 0.0205 Pa s and the resulting mean viscosity at a shear rate 768 s⁻¹ was 0.0086 Pa s.

It is intended that the specification and examples be considered as exemplary only, with a true scope and spirit of the present disclosure being indicated by the following claims.

The following aspects are preferred embodiments of the present application.
1. A method of preparing a gel for delivery to a target site of a patient, the method comprising:
   combining gellan gum and water to form a pre-mixture;
   heating the pre-mixture;
   adding a first salt comprising a monovalent cation and a second salt comprising a divalent cation to the pre-mixture to form a mixture;
   introducing the mixture into a reservoir; and
   cooling the mixture to form the gel inside the reservoir;
   wherein the gel is biocompatible and injectable from the reservoir through a needle to the target site.
2. The method of aspect 1, wherein the gel comprises 0.01% to 2.0% gellan gum by weight and/or 0.01 % to 0.1% by weight of the second salt, with respect to the total weight of the gel.
3. The method of aspect 1 or 2, wherein the mixture has a molar ratio of the monovalent cation to the divalent cation ranging from 5 to 200.
4. The method of any of the preceding aspects, wherein the monovalent cation of the first salt is sodium or potassium, and the divalent cation of the second salt is calcium or magnesium.
5. The method of any of the preceding aspects, wherein the first salt comprises sodium chloride or a hydrate thereof, and the second salt comprises calcium chloride or a hydrate thereof.
6. The method of any of the preceding aspects, wherein the gel has a viscosity ranging from 0.005 Pa s to 0.050 Pa s at a shear rate of 130 s⁻¹ and/or a viscosity ranging from 0.004 Pa·s to 0.010 Pa·s at a shear rate of 768 s⁻¹.
7. The method of any of the preceding aspects, wherein the mixture has an osmolality ranging from 240 mOsmol/kg to 340 mOsmol/kg.
8. The method of any of the preceding aspects, wherein the pre-mixture is heated at a temperature ranging from about 50°C to about 130°C.
9. he method of any of the preceding aspects, wherein the mixture has a temperature ranging from about 50°C to about 130°C when introduced into the reservoir.
10. The method of any of the preceding aspects, wherein the mixture is cooled to a temperature below about 50°C before introducing the mixture into the reservoir, the method further comprising heating the mixture at a temperature ranging from about 50°C to about 130°C while inside the reservoir.
11. The method of any of the preceding aspects, wherein the gel has a continuous, three-dimensional structure extending across an entire cross-sectional dimension of the reservoir.
12. The method of any of the preceding aspects, further comprising adding at least one coloring agent to the pre-mixture or the mixture.
13. The method of any of the preceding aspects, wherein the gel has an endotoxin level of 20 endotoxin units (EU) or less.
14. The method of any of the preceding aspects, wherein the reservoir is a barrel of a syringe or the reservoir is coupled to the needle via a flexible tube.
15. A medical device comprising the gel prepared according to the method of any of the preceding aspects.

## Claims

1. A medical device comprising:
a needle;
a reservoir coupled to the needle; and
a gel inside the reservoir, the gel comprising:
gellan gum;
a first salt comprising a monovalent cation;
a second salt comprising a divalent cation; and
water;
wherein the gel is biocompatible and injectable through the needle and
wherein the gel has a viscosity ranging from 0.004 Pa s to 0.010 Pa s at a shear rate of 768 s⁻¹.

2. The medical device of claim 1, wherein the monovalent cation of the first salt is sodium or potassium, and the divalent cation of the second salt is calcium or magnesium.

3. The medical device of claim 1, wherein the monovalent cation of the first salt is sodium.

4. The medical device of any of the preceding claims, wherein the first salt is sodium chloride.

5. The medical device of any of claims 1, 3, or 4, wherein the divalent cation of the second salt is calcium.

6. The medical device of any of the preceding claims, wherein the second salt is calcium chloride.

7. The medical device of any of the preceding claims, wherein a molar ratio of the first salt to the second salt in the gel ranges from 5 to 200.

8. The medical device of any of the preceding claims, wherein the gel has a continuous, three-dimensional structure extending across an entire cross-sectional dimension of the reservoir.

9. The medical device of any of the preceding claims, wherein the gel comprises 0.01% to 2.0% gellan gum by weight with respect to the total weight of the gel

10. The medical device of any of the preceding claims, wherein the gel has an endotoxin level of 20 endotoxin units (EU) or less.

11. The medical device of any of the preceding claims, wherein the gel further comprises at least one coloring agent.

12. The medical device of any of the preceding claims, wherein the gel further comprises brilliant blue coloring agent.

13. The medical device of any of the preceding claims, wherein the gel further comprises at least one sequestrant.

14. The medical device of any of the preceding claims, wherein the gel comprises 0.01 % to 0.1% by weight of the second salt with respect to a total weight of the gel.

15. The medical device of any of the preceding claims, wherein the reservoir is a barrel of a syringe.
